# EUROPÄISCHE PATENTSCHRIFT

(11) **EP 1 281 394 B1**
(45) Veröffentlichungstag und Bekanntmachung des Hinweises auf die Patenterteilung: **01.08.2007**
(21) Anmeldenummer: 02017285.4
(22) Anmeldetag: 01.08.2002
(51) Int. Cl.: A61Q 19/00, A61P 17/10

(54) **Kosmetische Verfahren**
Processes for cosmetics
Procédés cosmétiques

(30) Priorität: 02.08.2001 DE 10137466
(43) Veröffentlichungstag der Anmeldung: 05.02.2003
(73) Patentinhaber: Kuhlen, Marion, 52525 Heinsberg (DE)
(72) Erfinder: Dauven, Marlene, 52538 Gangelt-Stahe (DE)
(74) Vertreter: Bauer, Dirk

(56) Entgegenhaltungen:
- DE-A- 3 135 465
- DE-A- 19 801 471
- WOLFGAN RAAB; URSULA KINDL: "Pflegekosmetik, 3. Auflage" 1999 , WISS.VERL.-GES. , STUTTGART XP002262556 Seiten: 233-235, 243-246, 309, 310, 315, 316, 321-324

## Beschreibung

Die Erfindung betrifft ein Verfahren zu Aknebehandlung, Narbenbehandlung, Hautwiederherstellung bei Pigmentverschiebung, Hautwiederherstellung bei Couperose, Hautwiederherstellung bei Schwangerschaftsstreifen, zur Feuchtigkeitseinlagerung und Porenverkleinerung sowie Stoffwechselwiederherstellung mit Körpergewichtsreduzierung.

Derartige kosmetische Verfahren sind allgemein bekannt. Dabei kommen häufig die nachfolgend im einzelnen aufgeführten Verfahrensschritte, einzeln oder auch teilweise in bestimmten Kombinationen miteinander, zur Anwendung:

Mit Hilfe einer Peelingbehandlung wird die Haut von Hornschüppchen befreit, wodurch ein regulierender Einfluß auf die Talgproduktion ausgeübt und zudem überschüssiges Sebum von der Hautoberfläche abgenommen wird. Das Hautbild wird durch eine solche Maßnahme generell feiner und ebenmäßiger. Eine Peelingbehandlung, insbesondere, wenn sie als sogenanntes Enzympeeling durchgeführt wird, stellt eine intensive und dennoch schonende Tiefenreinigung der Haut dar. Vorzugsweise enthält ein Mittel zum Enzympeeling Hefeextrakt, Seidenpulver, Zitronenpulver, Saponin, Weizenkeimmehl und Weizengrieskleie als Wirkstoffe. Das Peeling wird mit lauwarmem Wasser angerührt, so daß eine cremige Paste entsteht. Mit einem Maskenpinsel wird es auf das gereinigte Gesicht, den Hals und das Dekolleté aufgetragen, wobei der Augenbereich ausgespart wird. Mit Hilfe einer warmen Kompresse wird das Peeling ca. 8 bis 10 Minuten feucht gehalten und kann einwirken. Anschließend wird das Peeling aufmassiert und mit reichlich Wasser abgenommen. Alternativ hierzu kann auch ein sogenanntes Körperpeeling angewendet werden, das auf der Kombination von Glykolsäure und rundgeschliffenen Granulaten basiert. Glykolsäure löst den Zellkitt in den oberen Hautschichten. Abgestorbene, trockene Hautschichten der Körperhaut werden abgetragen. Das Hautbild gewinnt hierdurch an Straffheit, wirkt feiner und geglättet. Weiter alternativ wird auch ein Ganzkörperpeeling mit Salzen durchgeführt. Ein hierbei verwendetes Produkt enthält fein gekörntes Meersalz sowie Trägeröle (z. B. Mandel- oder Avocadoöl) und ätherische Öle (Pfefferminze, Rosmarin und Lavendel. Durch die kleinen Salzkristalle werden trockene Hautschüppchen entfernt und der Hautstoffwechsel angeregt. Die ätherischen Öle entfalten eine revitalisierende Wirkung. Außerdem ist ein viertes Peelingverfahren bekannt, bei dem eine Mischung aus Reinigungsmilch, Abrasionskristallen (bestehend u. a. aus Aluminiumoxid) und Narbenöl verwendet wird. Die vier vorgenannten Arten der Peelingbehandlung werden nachfolgend jeweils als Spezialpeeling bezeichnet.

Mit Hilfe einer Hautausreinigung wird die Haut oftmals auf weitere Behandlungsschritte vorbereitet. Hierbei werden die durch Verhornungsstörungen hervorgerufenen Stauungen des Sebums angegangen, die Ausführungsgänge der Talgdrüsen werden geöffnet und der Sekretstau beseitigt. Es wird hierdurch verhindert, daß pathogene Keime in dem gestauten Talg eine Nahrungsgrundlage finden und es zu einer Entzündung der Talgdrüsengänge kommt.

Mit Hilfe einer sogenannten Farblichttherapie, auch Colortherapie genannt, wird die Wirkung des Lichtes auf das Resonanzgeschehen auf molekularer Ebene in der Zelle, d. h. die Wirkung der Photonen auf die in jedem höheren Organismen befindlichen Zellfarbstoffe (Zytochrome) ausgenutzt. Die Farblichttherapie nutzt dabei etablierte sinnesphysiologische Erkenntnisse über die Farbwirkung auf das menschliche Gehirn und damit auf alle immunologischen, hormonellen Prozesse. Sie benutzt auch zusätzlich unter Einbeziehung bestimmter Reflexbahnen grundlegendes neurologisches Wissen zur Behandlung vorn Organschäden. Ebenso ermöglicht sie den neueren Forschungsansatz über die großflächige Hautbestrahlung, insbesondere bei kosmetischen Problemen. Mit Hilfe geeigneter Vorrichtungen zur Farblichttherapie läßt sich die gesamte Farbskala des Lichtspektrums auf den menschlichen Körper leiten und die Energie erzeugende Kraft des Lichts, d.h. der Farben, durch verschiedene Behandlungsmöglichkeiten optimal nutzen. Möglich ist entweder eine punktförmige Konzentration oder eine flächige Streuung des Lichts. Alternativ ist eine kreisförmige oder lineare Bestrahlungsform möglich. Wählbar sind auch die Intensität und der richtige Farbton des einwirkenden Lichts.

Unter der Bezeichnung Vitalaser sind lampenartige Einrichtungen bekannt, die polarisiertes Licht aussenden. Die Wirkung von polarisiertem Licht auf den menschlichen Körper wurde im Zusammenhang mit der Erforschung von Laserstrahlen entdeckt. Seitdem wird polarisiertes Licht in zunehmendem Maße auch in der Kosmetologie angewandt. Die Wirkung dieses speziellen Lichts mit beträchtlicher Tiefenwirkung beruht darin, daß die Biostimulation der Zellen aktiviert, der Stoffwechsel angeregt und die Sauerstoffversorgung verbessert wird, und vermehrt Lymphozyten gebildet werden. Durch Zellaktivierung (Biostimulation) und verbesserte Sauerstoffzufuhr gewinnt altes Gewebe verstärkt seine Regenerationsfähigkeit zurück. Durch Vermehrung der Lymphozyten erfährt das Immunsystem eine Stärkung, wodurch sich die Infektionsanfälligkeit verringert. Die Aktivierung des Stoffwechsels entfaltet bei vielfältigen kosmetischen Problemstellungen hilfreiche Wirkungen und beugt des weiteren zahlreichen Erkrankungen und Altersprozessen entgegen. Vorteilhaft ist ein Polarisationsgrad von über 95% und eine spektrale Lichtverteilung, bei der 95 % der Ausgangsleistung im Bereich zwischen 400 und 200 nm liegen. Die Behandlungsdistanz liegt zwischen 5 und 15 cm und die Behandlungsdauer bei ca. 10 Minuten.

Bekannt ist es des weiteren, auf kosmetisch zu behandelnde Hautpartien eine sogenannte Mineralmaske aufzubringen. Diese besteht aus Betonit, Natriumchlorid, Zinkoxid und Magnesiumssulfat und dient zur Regeneration der Hautfunktionen auf natürlicher Basis. Durch die Mineralmaske wird eine bessere Aufnahme nachfolgender Mittel, eine Aktivierung des Hautstoffwechsels und eine verbesserte Lymphaktivität bewirkt. Das Ergebnis ist eine deutliche Vitalisierung, Glättung und Elastizitätszunahme der Haut.

Zur kosmetischen Behandlung von Akne ist die Verwendung von Aknetinkturen bekannt. Derartige Tinkturen enthalten beispielsweise Pirocton, Olamine, Salicylsäure, Süßholzwurzelextrakt, Flechtenextrakt, Heilerde und Zinkoxid. Süßholzwurzelextrakt und Flechtenextrakt wirken bakteriostatisch und lassen entzündliche Unreinheiten schneller abklingen. Heilerde und Zinkoxid haben eine absorbierende Wirkung, so daß insgesamt Pickel und unreine Haut schnell und mit nachhaltiger Wirkung kosmetisch behandelt werden können. Insgesamt wirkt die Aknetinktur schmerzstillend, desinfizierend sowie lindernd bei Blutandrang und Juckreiz.

Ein weitere Behandlungsmaßnahme besteht in der Anwendung eines sogenannten Flächenlasers. Dabei handelt es sich um einen Infrarot-Laser (auch Soft-Laser genannt) mit einer Mehrzahl (z. B. 9) gleichzeitig arbeitenden Laserdioden, mit denen gleichzeitig eine größere Hautfläche (ca. 100 mm²) bestrahlt werden kann. Der Abstand zur Haut bei der Bestrahlung beträgt ca. 3 mm.

Bekannt ist des weiteren die örtliche Abrasion der Haut, um hierdurch Hautveränderungen und Narben beseitigen zu können. Abrasionsgeräte besitzen in der Regel integrierte Vakuumpumpen, die sterilisierte Mikrokristalle ansaugen, die anschließend mit hoher Geschwindigkeit auf die Haut auftreffen und Gewebepartikel herauslösen. Ein zweiter Schlauch saugt diese herausgelösten Gewebepartikel und die verbrauchten Kristalle ab und leitet sie einem Entsorgungsgefäß zu. Durch den Aufprall der Kristalle entstehen viele mikroskopische Läsionen im Gewebe, die die gesunden Zellen zu erhöhter Teilung anregen. Die neuen Epithelzellen wandern in den behandelten Hautbereich ein und ersetzen nach jeder Behandlung das abgetragene Gewebe mehr und mehr mit normaler Haut. Gleichzeitig erfolgt eine Mikromassage, wodurch die Haut spürbar gestrafft wird. Die Zahl der Behandlungen sowie die anschließenden Pausen (3 bis 7 Tage) hängen von der Narben- und Hautbeschaffenheit und der Intensität ab. Die Intensität kann über das regulierbare Vakuum und/oder über die Geschwindigkeit, mit der der Behandlungskopf über die Haut geführt wird, eingestellt werden.

Ferner bildet die Ozontherapie einen bekannten Baustein in der kosmetischen Hautbehandlung. Hierbei wird Ozon innerhalb eines über die Haut geführten Behandlungskopfs erzeugt, wobei auf die zu behandelnden Bereiche zuvor ein Öl oder eine Creme appliziert wird. Das innerhalb des Behandlungskopfs durch Entladungen in einem elektromagnetischen Feld gebildete Ozon dringt in die Haut ein und beschleunigt den Vorgang der Feuchtigkeitseinlagerung.

Zum Stand der Technik zählen auch sogenannte Feuchtigkeitsmasken, mit deren Hilfe in der Haut ein Feuchtigkeitsdepot angelegt werden soll. Durch Wirkstoffkombinationen aus Algen-, Pflanzen- und Fruchtextrakten wird die Hautoberfläche geglättet, die Gesichtskontur verbessert und das hauteigene Schutzsystem aktiviert. Die Haut gewinnt an Geschmeidigkeit und an Elastizität. Typische Wirkstoffe sind hier CODIUM TOMENTOSUM, (ALGAE)EXTRACT, LOCUST BEAN (CERATONIA SILIQUA) GUM HYDROLIZED CA-DEIN, Macadamianußöl, Aprikosenextrakt und Ananaspulver.

Auch mit Hilfe einer sogenannten Alphamassage lassen sich vielfältige kosmetische Effekte erzielen. Mit Hilfe von Alphamassagegeräten läßt sich der gesamte Körper unter trockener Hitze bei einstellbarer Temperatur baden. Mit Hilfe von Motoren läßt sich eine pulsierende Wellenmassage mit individuell steuerbarer Vibration und Stärke realisieren. Integrierte UV-Lampen erzeugen aktiven Sauerstoff zur Desinfektion der Haut. Ionisierte Gesichtsluft kühlt und erfrischt das Gesicht. Von dem Gerät abgesandte Alphawellen sorgen für geistige und körperliche Entspannung. Eine Spezialbrille mit Lichtreflexen und wahlweise Kopfhörern synchronisiert Licht und Geräusch auf die individuell vorgegebene Wellenlänge für den Zustand optimaler mentaler Fitneß.

Mit Hilfe eines Kollagenvlieses läßt sich das Hautgleichgewicht auch bei sehr sensibler und reizempfindlicher Haut wieder herstellen. Eine derartige Behandlung wird in der Regel als Kurbehandlung bei entfeuchteter Haut in einer Kabine durchgeführt. Ein Vlies wird hierzu kopfformgerecht zugeschnitten und mit einer Öffnung in Nasen- und Mundpartie freigelassen. Die Augenpartie kann bei gewissen Vliesarten mit in die Behandlung einbezogen werden.

Das Vlies wird mit einem wasserdurchtränkten Schwamm angefeuchtet, so daß es glatt auf der Haut aufliegt. Nach 10 bis 20 Minuten wird das Vlies einfach vom Gesicht abgezogen. Der Wirkstoff eines derartigen Vlieses besteht in der Regel aus 100% nativem Kollagen. Hierdurch wird die Haut intensiv mit Feuchtigkeit versorgt, das Gewebe gestrafft und Fältchen geglättet.

Mit Hilfe einer gleichfalls bekannten Sauerstoffbehandlung wird der Hautstoffwechsel und die Qualität der Hautzellen verbessert. Die durch Aufbringen geeigneter Präparate realisierten Sauerstoffbehandlungen können in Form einer separaten Tages- oder Nachtpflege oder in kombinierten Maßnahmen bestehen. Ausschlaggebend ist hier die Art des Hauttyps. Häufig verwendete Wirkstoffe sind Weizenkeimöl, Peptidkomplexe, Kamille, Hagebutte, Antiphlogisticum, Calendulaöl, Avocadoöl und Haselnußöl, Allantoin, Proteinderivate, Frauenmantel, Bisabolol, Aloe Vera und Mandelöl.

Bekannt sind des weiteren Infrarotbehandlungen, die mit Hilfe von Geräten zur Wärmebestrahlung von Gesicht und Körper durchgeführt werden. Der Strahlungsabstand beträgt hier ca. 40 bis 60 cm bei einer Bestrahlungsdauer von ca. 30 Minuten.

In der Kosmetologie werden des weiteren sogenannte Dynalineverfahren angewendet, mit denen Analgesieeffekte, Muskelkontraktionen, Nervenstimulation und Einleitung biochemischer Prozesse erreicht werden sollen. Hierzu werden großflächige Elektrodenmatten mit bis zu 32 Einzelelektroden bzw. 16 Elektrodenpaaren eingesetzt, die auf Hautpartien der zu behandelnden Person aufgelegt werden. Aufgrund der individuellen Ansteuerbarkeit und Vernetzbarkeit der Elektroden lassen sich sogenannte Gleitwellen erzeugen, mit denen eine zusammenhängende Stimulation ganzer Muskelgruppen möglich ist. Weitere Merkmale sind eine integrierte, zuschaltbare Wärme sowie die Mischbarkeit der Impulsarten. Bei den abgegebenen Signalen handelt es sich um bipolare Impulse im Nieder- bis Mittelfrequenzbereich ohne Gleichspannungsanteil. Bei den Impulsformen kann zwischen einer Dreieck-, Exponential- und Rechteckform gewählt werden. Im Ergebnis liegt somit eine intensive, als sehr angenehm empfundene Elektromassage vor. Die insbesondere behandelten Körperpartien sind Nacken- und Schultermuskulatur, obere Extremitäten, Rumpfmuskulatur, Darm und Bauch, Hüfte sowie untere Extremitäten. Anwendungsfälle für die Dynalineverfahren ist eine Cellulitebehandlung, die Beseitigung von natürlichen Lymphenstauungen, substantieller Muskelaufbau, gezielte Gewichtsabnahme sowie Haut- und Gewebestraffung sowie eine wirksame Entspannung der Muskulatur.

Mit Hilfe einer Lymphdrainage - die vorzugsweise mit dem Herzschlagrhythmus synchronisiert sein sollte - wird das Gewebe entschlackt und die Stoffwechselvorgänge angeregt. Bekannte Geräte sind mit mehreren Luftkammern in Stiefeln, Oberschenkelmanschetten, Bauch-und Pomanschetten versehen. Die Luftkammern werden zeitlich aufeinander abgestimmt im Herzrhythmus aufgepumpt und üben so einen gezielten Druck auf das Gewebe aus. Hierdurch wird der Lymphfluß beschleunigt und aktiviert.

Der Erfindung liegt die Aufgabe zugrunde, diverse kosmetische Behandlungsverfahren vorzuschlagen, mit denen sich durch ganz bestimmte Kombinationen bekannter Einzelkomponenten besonders vorteilhafte Wirkungen bei der Behandlung von Akne und Narben, der Hautwiederherstellung bei Pigmentverschiebungen, bei Couperose und bei Schwangerschaftsstreifen, bei der Feuchtigkeitseinlagerung mit Porenverfeinerung sowie der Stoffwechselwiederherstellung mit Körpergewichtsreduzierung erzielen lassen.

Bei den nachfolgend erläuterten Verfahren sind unter den einzelnen schlagwortartig benannten Verfahrensschritten solche Vorgehensweisen zu verstehen, wie sie in der vorangegangenen Beschreibung des Standes der Technik näher erläutert wurden.

In bezug auf die Aknebehandlung wird die zugrunde liegende Aufgabe durch ein kosmetisches Verfahren mit den nachfolgend aufgeführten Verfahrensschritten gelöst:
a) Spezialpeeling
b) Ausreinigung der Haut
c) Farblichttherapie
d) Vitalaserbehandlung
e) Mineralmaske
f) Aknetinktur
g) Flächenlaserbehandlung.

Vorzugsweise werden die vorgenannten Verfahrensschritte in der vorstehend wiedergegebenen Reihenfolge ausgeführt. Alternativ hierzu ist jedoch auch die Anwendung des Vitalasers vor der Farblichttherapie möglich.

Nach einer Ausgestaltung dieses Verfahrens wird nach der Applikation der Aknetinktur auf die betroffenen Hautflächen eine sogenannte Skinpurancreme aufgetragen, die als Wirkstoffe Chlorhexidindigluconat, Süßholzwurzelextrakt, Glukose-Pentaacetat, Sebumine SB 12^{®} und bedarfsweise D-Panthenol, Jojobaöl, Sheabutter und Perhydrosqualen sowie Bisabolol enthält.

Ein erfindungsgemäßes kosmetisches Verfahren zur Narbenbehandlung zeichnet sich durch die folgenden Merkmale aus:
a) Spezialpeeling
b) Ausreinigung der Haut
c) Abrasion der Haut
d) Farblichttherapie
e) Vitalaserbehandlung
f) Mineralmaske
g) Flächenlaserbehandlung
h) Ozontherapie.

Gemäß einer Ausgestaltung dieses Verfahrens werden die einzelnen Verfahrensschritte in der vorstehend wiedergegebenen Reihenfolge durchgeführt. Alternativ hierzu kann die Reihenfolge der Verfahrensschritte d) (Farblichttherapie) und e) (Vitalaser) vertauscht werden.

Die Erfindung weiter ausgestaltend wird vorgeschlagen, daß nach der Mineralmaske (Verfahrensschritt f)) je nach Hauttyp eine Feuchtigkeitsbehandlung, z. B. eine Vitamin-Honigmaske, aufgebracht wird. Diese Maske wird ca. 1 bis 2 mm dick aufgetragen und ca. 10 Minuten zur Einwirkung auf der Haut belassen und anschließend mit einem Kosmetikschwämmchen oder einer Kompresse und warmem Wasser abgenommen. Die Vitamin-Honigmaske enthält als Wirkstoffe Conjugated Glycopolypeptids, Honig, Weizenkeimöl, Vitamin E und Vitamin A. Durch diese Maßnahme wird die Haut vitalisiert und geglättet und der Sauerstoffumsatz verbessert. Außerdem wird der Hautstoffwechsel und die Mikrozirkulation der Haut aktiviert.

Des weiteren wird als Ergänzung vorgeschlagen, als letzten Verfahrensschritt Aromaessenzen, d. h. ätherische Öle, äußerlich auf den betroffenen Hautstellen anzuwenden. Zur Narbenbehandlung eignen sich insbesondere Matricaria chamomilla, Lavandula officinalis, Pelargonium graveolens, Citrus aurantium var. dulcis und Melaleuca alternifolia.

Das erfindungsgemäße kosmetische Hautwiederherstellungsverfahren bei Pigmentverschiebungen zeichnet sich durch die folgenden Verfahrensschritte aus:
a) Spezialpeeling
b) Ausreinigung der Haut
c) Abrasion der Haut
d) Farblichttherapie
e) Vitalaserbehandlung
f) Mineralmaske
g) Feuchtigkeitsmaske
h) Flächenlaserbehandlung
i) Ozontherapie.

Nach einer besonders bevorzugten Ausgestaltung werden die vorgenannten Verfahrensschritte in der genannten Reihenfolge hintereinander ausgeführt. Um die Wirkung zu verbessern, kann die Ozontherapie mit einer Anwendung von ätherischen Ölen kombiniert werden.

Mit dem erfindungsgemäßen Hautwiederherstellungsverfahren lassen sich insbesondere Pigmentverschiebungen und Altersflecken wirkungsvoll beseitigen.

Die Erfindung umfaßt des weiteren ein kosmetisches Hautwiederherstellungsverfahren bei Couperose. Hierbei handelt es sich um geplatzte Äderchen, die oftmals als unschön betrachtet werden. Das erfindungsgemäße Verfahren umfaßt die folgenden Verfahrensschritte.
j) Spezialpeeling
a) Farblichttherapie

Die Reihenfolge der beiden vorgenannten Verfahrensschritte sollte unbedingt eingehalten werden, um den gewünschten Erfolg zu garantieren. Bedarfsweise können die beiden vorgenannten Verfahrensschritte durch eine Behandlung mittels eines Vitalaser als dritten Verfahrensschritt ergänzt werden. Zusätzlich ist die Anwendung von Ampullen zur Couperosebehandlung angezeigt.

Nach der Erfindung wird des weiteren ein kosmetisches Hautwiederherstellungsverfahren bei Schwangerschaftsstreifen mit den folgenden Verfahrensschritten vorgeschlagen:
a) Spezialpeeling
b) Abrasion der Haut
c) Farblichttherapie
d) Vitalaserbehandlung
e) Alphamassage des gesamten Körpers
f) Ozonbehandlung
g) Flächenlaserbehandlung

Vorzugsweise werden die vorgenannten Verfahrensschritte in der vorstehend wiedergegebenen Reihenfolge ausgeführt. Zusätzlich können noch eine Feuchtigkeitsmaske und/oder andere Präparate zur Hautwiederherstellung (Vitaminpräparate o. ä.) aufgebracht werden.

Ergänzend hierzu ist es sinnvoll, die Alphamassage mit einer Ganzkörper-Mineralbehandlung oder einer Ganzkörper-Algenbehandlung zu kombinieren.

Je nach Hauttyp können des weiteren insbesondere eine Feuchtigkeitscreme und ein Hautfunktionsöl angezeigt sein.

Das erfindungsgemäße Verfahren zur Feuchtigkeitseinlagerung und Porenverkleinerung umfaßt die folgenden Schritte:
a) Spezialpeeling
b) Ausreinigen der Haut
c) Abrasion der Haut
d) Farblichttherapie
e) Vitalaserbehandlung
f) Thermomaskenbehandlung
g) Kollagenvliesbehandlung
h) Sauerstoffbehandlung
i) Ozonbehandlung
j) Flächenlaserbehandlung

Vorzugsweise werden die vorstehend genannten Verfahrensschritte in der zuvor wiedergegebenen Reihenfolge ausgeführt.

Sinnvollerweise kann die Thermomaskenbehandlung mit ätherischen Ölen, z.B. Aloe Vera-Masken, ergänzt werden. Auch ist es denkbar, verschiedene Masken als Unterlagen für die Thermomaske zu verwenden.

Schließlich wird erfindungsgemäß noch ein kosmetisches Stoffwechselwiederherstellungsverfahren mit Körpergewichtsreduzierung vorgeschlagen, das sich durch die folgende Verfahrensweise auszeichnet:

| | |
|---|---|
| 1. Tag: | Infrarotbehandlung für ca. 20 bis 40 Minuten, Dynalinebehandlung für ca. 40 bis 60 Minuten Lymphdrainage für ca. 20 bis 40 Minuten |
| 3. Tag: | Infrarotbehandlung für 20 bis 40 Minuten Alphamassage für 50 - 70 Minuten Lymphdrainage für 20 bis 40 Minuten |
| 5. Tag: | Infrarotbehandlung für 20 bis 40 Minuten Dynalinebehandlung für 40 bis 60 Minuten Lymphdrainage für 20 bis 40 Minuten |

Wird eine derartige Behandlungsweise mit einer Ernährungsberatung kombiniert, so ergibt sich eine spürbare Verbesserung der Stoffwechselqualität, so daß Gewichtsreduzierungen ohne den berüchtigten Jojo-Effekt möglich sind.

## Patentansprüche

1. Kosmetisches nich-therapeutisches Verfahren zur Behandlung unreiner Haut mit den nachfolgenden Verfahrensschritten:
a) Spezialpeeling
b) Ausreinigung der Haut
c) Farblichttherapie
d) Vitalaserbehandlung
e) Mineralmaske
f) Aknetinktur
g) Flächenlaserbehandlung.

2. Verfahren nach Anspruch 1, **dadurch gekennzeichnet, daß** die Verfahrensschritte in der in Anspruch 1 genannten Reihenfolge durchgeführt werden.

3. Verfahren nach Anspruch 1 oder 2, **dadurch gekennzeichnet, daß** im Anschluß an die Aknetinktur eine Purancreme auf die zu behandelnden Hautstellen aufgebracht wird.

4. Kosmetisches nicht-therapeutisches Verfahren zur Narbenbehandlung mit den nachfolgend aufgeführten Verfahrensschritten:
a) Spezialpeeling
b) Ausreinigen der Haut
c) Abrasion der Haut
d) Farblichttherapie
e) Vitalascrbehandlung
f) Mineralmaske
g) Flächenlaserbehandlung
h) Ozontherapie.

5. Verfahren nach Anspruch 4, **dadurch gekennzeichnet, daß** die einzelnen Verfahrensschritte in der in Anspruch 4 genannten Reihenfolge ausgeführt werden.

6. Verfahren nach Anspruch 4 oder 5, **dadurch gekennzeichnet, daß** im Anschluß an die Mineralmaske eine Vitamin-Honigmaske auf die zu behandelnden Hautstellen aufgebracht wird.

7. Kosmetisches nicht therapeutisches Verfahren zur Hautwiederherstellung bei Pigmentverschiebung mit den nachfolgend aufgeführten Verfahrensschritten:
a) Spezialpeeling
b) Ausreinigen der Haut
c) Abrasion der Haut
d) Farblichttherapie
e) Vitalaserbehandlung
f) Mineralmaske
g) Feuchtigkeitsmaske
h) Flächenlaserbehandlung
i) Ozontherapie.

8. Verfahren nach Anspruch 7, **dadurch gekennzeichnet, daß** die einzelnen Verfahrensschritte der in Anspruch 7 angegebenen Reihenfolge ausgeführt werden.

9. Kosmetisches nicht therapeutisches Verfahren zur Hautwiederherstellung bei Couperose mit den nachfolgend aufgeführten Verfahrensschritten:
a) Spezialpeeling
b) Farblichttherapie.

10. Verfahren nach Anspruch 9, **dadurch gekennzeichnet, daß** die Verfahrensschritte in der in Anspruch 9 angegebenen Reihenfolge durchgeführt werden.

11. Verfahren nach Anspruch 9 oder 10, **dadurch gekennzeichnet, daß** im Anschluß an die Farblichttherapie eine Vitalaserbehandlung durchgeführt wird.

12. Kosmetisches Verfahren zur Hautwiederherstellung bei Schwangerschaftsstreifen mit den nachfolgend aufgeführten Verfahrensschritten:
a) Spezialpeeling
b) Abrasion der Haut
c) Farblichttherapie
d) Vitalaserbehandlung
e) Alphamassage des gesamten Körpers
f) Ozontherapie
g) Flächenlaserbehandlung.

13. Verfahren nach Anspruch 12, **dadurch gekennzeichnet, daß** die einzelnen Verfahrensschritte in der in Anspruch 12 wiedergegebenen Reihenfolge ausgeführt werden.

14. Verfahren nach Anspruch 12 oder 13, **dadurch gekennzeichnet, daß** die Alphamassage mit einer Mineralbehandlung oder einer Algenbehandlung des gesamten Körpers kombiniert wird.

15. Kosmetisches Verfahren zur Feuchtigkeitseinlagerung und Porenverkleinerung mit den nachfolgend aufgeführten Verfahrensschritten:
a) Spezialpeeling
b) Ausreinigung der Haut
c) Abrasion der Haut
d) Farblichttherapie
e) Vitalaserbehandlung
f) Thermomaskenbehandlung
g) Kollagenvliesbehandlung
h) Sauerstoffbehandlung
i) Ozontherapie
j) Flächenlaserbehandlung.

16. Verfahren nach Anspruch 15, **dadurch gekennzeichnet, daß** die einzelnen Verfahrensschritte in der in Anspruch 15 wiedergegebenen Reihenfolge ausgeführt werden.

17. Verfahren nach Anspruch 15 oder 16, **dadurch gekennzeichnet, daß** für die Thermomaskenbehandlung weitere Masken als Unterlage verwendet werden.

18. Kosmetisches nich-therapeutisches Verfahren zur Stoffwechselwiederherstellung mit Körpergewichtsreduzierung mit den folgenden Verfahrensschritten:
| | |
|---|---|
| 1. Behandlungstag: | a) Infrarotbehandlung für 20 bis 40 Minuten |
| | b) Dynalinebehandlung für 40 bis 60 Minuten |
| | c) Lymphdrainage für 20 bis 40 Minuten |
| 3. Behandlungstag: | a) Infrarotbehandlung für 20 bis 40 Minuten |
| | b) Alphamassage für 50 bis 70 Minuten |
| | c) Lymphdrainage für 20 bis 40 Minuten |
| 5. Behandlungstag: | a) Infrarotbehandlung für 20 bis 40 Minuten |
| | b) Dynalinebehandlung für 40 bis 60 Minuten |
| | c) Lymphdrainage für 20 bis 40 Minuten |
wobei die Behandlung am 7., 11., 15. und 19. Behandlungstag der Behandlung am 3. Behandlungstag, und die Behandlung am 9., 13. und 17. Behandlungstag der Behandlung am 1. bzw. 5. Behandlungstag entspricht, und wobei an gradzahligen Tagen keine Behandlungen durchgeführt werden.

19. Verfahren nach einem oder mehreren der Ansprüche 1 bis 18, **dadurch gekennzeichnet, daß** ergänzend ätherische Öle auf die zu behandelnden Stellen der Haut appliziert bzw. in der Atemluft zerstäubt werden.

## Claims

1. Cosmetic non-therapeutic method for treating unclear skin comprising the following method steps:
a) special peeling
b) cleansing the skin
c) coloured light therapy
d) vital laser treatment
e) mineral mask
f) acne tincture
g) superficial laser treatment.

2. Method according to claim 1, **characterised in that** the method steps are carried out in the sequence mentioned in claim 1.

3. Method according to claim 1 or 2, **characterised in that**, following the acne tincture, a puran cream is applied to the locations on the skin to be treated.

4. Cosmetic non-therapeutic method for scar treatment comprising the method steps listed below:
a) special peeling
b) cleansing the skin
c) skin abrasion
d) coloured light therapy
e) vital laser treatment
f) mineral mask
g) superficial laser treatment
h) ozone therapy.

5. Method according to claim 4, **characterised in that** the individual method steps are carried out in the sequence mentioned in claim 4.

6. Method according to claim 4 or 5, **characterised in that**, following the mineral mask, a vitamin-honey mask is applied to the locations of the skin to be treated.

7. Cosmetic non-therapeutic method for restoring the skin in the case of pigment displacement comprising the method steps listed below:
a) special peeling
b) cleansing the skin
c) skin abrasion
d) coloured light therapy
e) vital laser treatment
f) mineral mask
g) moisture mask
h) superficial laser treatment
i) ozone therapy.

8. Method according to claim 7, **characterised in that** the individual method steps are carried out in the sequence disclosed in claim 7.

9. Cosmetic non-therapeutic method for restoring the skin in the case of couperose comprising the method steps listed below:
a) special peeling
b) coloured light therapy.

10. Method according to claim 9, **characterised in that** the method steps are carried out in the sequence disclosed in claim 9.

11. Method according to claim 9 or 10, **characterised in that**, following the coloured light therapy, a vital laser treatment is carried out.

12. Cosmetic method for restoring the skin in the case of stretch marks comprising the method steps listed below:
a) special peeling
b) skin abrasion
c) coloured light therapy
d) vital laser treatment
e) alpha massage of the whole body
f) ozone therapy
g) superficial laser treatment.

13. Method according to claim 12, **characterised in that** the individual method steps are carried out in the sequence reproduced in claim 12.

14. Method according to claim 12 or 13, **characterised in that** the alpha massage is combined with a mineral treatment or an algae treatment of the whole body

15. Cosmetic method for moisture incorporation and pore reduction comprising the method steps listed below:
a) special peeling
b) cleansing the skin
c) skin abrasion
d) coloured light therapy
e) vital laser treatment
f) thermo mask treatment
g) collagen fleece treatment
h) oxygen treatment
i) ozone therapy
j) superficial laser treatment.

16. Method according to claim 15, **characterised in that** the individual method steps are carried out in the sequence reproduced in claim 15.

17. Method according to claim 15 or 16, **characterised in that** further masks are used as an underlay for the thermo mask treatment.

18. Cosmetic non-therapeutic method for restoring the metabolism with body weight reduction comprising the following method steps:
| | |
|---|---|
| 1^{st} treatment day: | a) infrared treatment for 20 to 40 minutes |
| | b) Dynaline treatment for 40 to 60 minutes |
| | c) lymph drainage for 20 to 40 minutes |
| 3^{rd} treatment day: | a) infrared treatment for 20 to 40 minutes |
| | b) alpha massage for 50 to 70 minutes |
| | c) lymph drainage for 20 to 40 minutes |
| 5^{th} treatment day: | a) infrared treatment for 20 to 40 minutes |
| | b) Dynaline treatment for 40 to 60 minutes |
| | c) lymph drainage for 20 to 40 minutes |
wherein the treatment on the 7^{th}, 11^{th}, 15 ^{th} and 19^{th} treatment day corresponds to the treatment on the 3^{rd} treatment day, and the treatment on the 9^{th}, 13^{th} and 17^{th} treatment day corresponds to the treatment on the 1^{st} and 5^{th} treatment day, and wherein on even-numbered days no treatments are carried out.

19. Method according to any one or more of claims 1 to 18, **characterised in that**, in addition, essential oils are applied to the locations of the skin to be treated or are sprayed in the air that is breathed.

## Revendications

1. Procédé cosmétique non thérapeutique pour le traitement des impuretés cutanées, comprenant les étapes de procédé suivantes:
a) gommage spécial
b) dépuration de la peau
c) chromophotothérapie
d) traitement par laser froid
e) masque minéral
f) teinture contre l'acné
g) traitement de surface au laser.

2. Procédé selon la revendication 1, **caractérisé en ce que** les différentes étapes du procédé sont exécutées dans l'ordre indiqué dans la revendication 1.

3. Procédé selon la revendication 1 ou 2, **caractérisé en ce que** la teinture contre l'acné est suivie d'une application de crème Puran sur les zones de peau à traiter.

4. Procédé cosmétique non thérapeutique pour le traitement des cicatrices, comprenant les étapes de procédé suivantes :
a) gommage spécial
b) dépuration de la peau
c) dermabrasion
d) chromophotothérapie
e) traitement par laser froid
f) masque minéral
g) traitement de surface au laser
h) ozonethérapie.

5. Procédé selon la revendication 4, **caractérisé en ce que** les différentes étapes du procédé sont exécutées dans l'ordre indiqué dans la revendication 4.

6. Procédé selon la revendication 4 ou 5, **caractérisé en ce que** le masque minéral est suivi de l'application d'un masque au miel vitaminé sur les zones de peau à traiter.

7. Procédé cosmétique non thérapeutique pour la régénération cutanée en cas de dépigmentation, comprenant les étapes de procédé suivantes:
a) gommage spécial
b) dépuration de la peau
c) dermabrasion
d) chromophotothérapie
e) traitement par laser froid
f) masque minéral
g) masque hydratant
h) traitement de surface au laser
i) ozonethérapie.

8. Procédé selon la revendication 7, **caractérisé en ce que** les différentes étapes du procédé sont exécutées dans l'ordre indiqué dans la revendication 7.

9. Procédé cosmétique non thérapeutique pour la régénération cutanée en cas de couperose, comprenant les étapes de procédé suivantes:
a) gommage spécial
b) chromophotothérapie.

10. Procédé selon la revendication 9, **caractérisé en ce que** les différentes étapes du procédé sont exécutées dans l'ordre indiqué dans la revendication 9.

11. Procédé selon la revendication 9 ou 10, **caractérisé en ce que** la chromophotothérapie est suivie d'un traitement par laser froid.

12. Procédé cosmétique non thérapeutique pour la régénération cutanée en cas de vergetures, comprenant les étapes de procédé suivantes :
a) gommage spécial
b) dermabrasion
c) chromophotothérapie
d) traitement par laser froid
e) massage alpha du corps entier
f) ozonethérapie
g) traitement de surface au laser.

13. Procédé selon la revendication 12, **caractérisé en ce que** les différentes étapes du procédé sont exécutées dans l'ordre indiqué dans la revendication 12.

14. Procédé selon la revendication 12 ou 13, **caractérisé en ce que** le massage alpha est combiné avec un soin aux minéraux ou un soin aux algues du corps entier.

15. Procédé cosmétique non thérapeutique pour l'accumulation d'hydratation et la réduction des pores, comprenant les étapes de procédé suivantes :
a) gommage spécial
b) dépuration de la peau
c) dermabrasion
d) chromophotothérapie
e) traitement par laser froid
f) traitement par masque thermique
g) traitement par feutre de collagène
h) oxygénothérapie
i) ozonethérapie
j) traitement de surface au laser.

16. Procédé selon la revendication 15, **caractérisé en ce que** les différentes étapes du procédé sont exécutées dans l'ordre indiqué dans la revendication 15.

17. Procédé selon la revendication 15 ou 16, **caractérisé en ce que** d'autres masques sont utilisés comme couche sous-jacente pour le traitement par masque thermique.

18. Procédé cosmétique non thérapeutique pour le rétablissement du métabolisme avec réduction du poids corporel, comprenant les étapes de procédé suivantes :
| | |
|---|---|
| 1^{er} jour de traitement : | a) traitement aux infrarouges pendant 20 à 40 minutes |
| | b) soin Dynaline pendant 40 à 60 minutes |
| | c) drainage lymphatique pendant 20 à 40 minutes |
| 3^{ème} jour de traitement : | a) traitement aux infrarouges pendant 20 à 40 minutes |
| | b) massage alpha pendant 50 à 70 minutes |
| | c) drainage lymphatique pendant 20 à 40 minutes |
| 5^{ème} jour de traitement : | a) traitement aux infrarouges pendant 20 à 40 minutes |
| | b) soin Dynaline pendant 40 à 60 minutes |
| | c) drainage lymphatique pendant 20 à 40 minutes. |
le traitement au 7^{ème}, 11^{ème}, 15^{ème} et 19^{ème} jour de traitement correspondant à celui du troisième jour de traitement, et le traitement au 9^{ème}, 13^{ème} et 17^{ème} jour de traitement au traitement du premier ou du cinquième jour, et aucun traitement n'étant exécuté les jours pairs.

19. Procédé selon une ou plusieurs des revendications 1 à 18, **caractérisé en ce qu'**en complément, des huiles essentielles sont appliquées sur la peau dans les zones à traiter ou pulvérisées dans l'air respiré.
